(19) Europäisches Patentamt
European Patent Office
Office européen des brevets

(11) **EP 2 192 380 A2**

(12) **EUROPÄISCHE PATENTANMELDUNG**

(43) Veröffentlichungstag:
**02.06.2010 Patentblatt 2010/22**

(51) Int Cl.:
*G01B 21/04* (2006.01)     *G01N 23/04* (2006.01)

(21) Anmeldenummer: **10002412.4**

(22) Anmeldetag: **24.05.2005**

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **26.05.2004 DE 102004026357**
**14.10.2004 DE 102004050257**
**20.04.2005 DE 102005018447**

(62) Dokumentnummer(n) der früheren Anmeldung(en) nach Art. 76 EPÜ:
**05750966.3 / 1 749 190**

(71) Anmelder: **Werth Messtechnik GmbH**
**35394 Giessen (DE)**

(72) Erfinder:
• **Christoph, Ralf, Dr.**
**35394 Gießen (DE)**
• **Rauh, Wolfgang, Dr.**
**35094 Lahntal-Goßfelden (DE)**

(74) Vertreter: **Stoffregen, Hans-Herbert**
**Patentanwalt**
**Friedrich-Ebert-Anlage 11b**
**63450 Hanau (DE)**

Bemerkungen:
Diese Anmeldung ist am 09-03-2010 als Teilanmeldung zu der unter INID-Code 62 erwähnten Anmeldung eingereicht worden.

(54) **Verfahren zum Messen eines Objektes mit einem Koordinatenmessgerät sowie Koordinatenmessgerät**

(57) Die Erfindung bezieht sich auf ein Koordinatenmessgerät (110) zum Messen eines Objektes (3) mit einer Röntgensensorik umfassend eine Röntgenstrahlenquelle (10) und zumindest einen die Röntgenstrahlen erfassenden Röntgenstrahlensensor (7), die in x-, y- und/ oder z-Richtung des Koordinatenmessgerätes relativ zu dem Objekt positionierbar ist. Um auch Messobjekte größerer Ausdehnung problemlos messen zu können, wird vorgeschlagen, dass die Röntgensensorik (7, 10) in dem Koordinatenmessgerät (10) positionierbar ist.

Fig. 1

EP 2 192 380 A2

**Beschreibung**

Die Erfindung bezieht sich auf ein Verfahren zum Messen eines Objektes mit einem Koordinatenmessgerät mit einer Röntgensensorik umfassend eine Röntgenstrahlquelle und zumindest einen die Röntgenstrahlen erfassenden Röntgenstrahlensensor. Auch nimmt die Erfindung Bezug auf ein Koordinatenmessgerät zum Messen eines Objektes mit einer Röntgensensorik als erste Sensorik umfassend eine Röntgenstrahlenquelle, zumindest einen die Röntgenstrahlen erfassenden Röntgenstrahlensensor.

Zum Messen der Geometrie von Werkstücken sind Koordinatenmessgeräte mit verschiedenen Sensoren bekannt. Als solche Sensoren werden optische und taktile Sensoren beschrieben (DE.Z.: Die Bibliothek der Technik, Bd. 248). Ebenfalls ist der Einsatz von Computer-Tomographen zur Bestimmung von Werkstückgeometrien insbesondere von Fehlstellen bekannt. So wird in der DE-A- 103 31 419 eine Kombination beider Geräte beschrieben. Hierbei ist ein Computer-Tomograph fest am Grundaufbau des Koordinatenmessgerätes befestigt. Dabei wird mittels klassischer Koordinatenmesstechnik-Sensorik die Position des Messobjektes bestimmt und hierauf folgend in den Messbereich des Computer-Tomographen positioniert.

Beim beschriebenen Stand der Technik finden verschiedene Aufgabenstellungen keine Beachtung. Ungelöst bleibt zum Beispiel das Problem, dass die Messobjekte eine größere Ausdehnung aufweisen können als der Messbereich des Computer-Tomographen. Da dieser starr am Grundaufbau des Koordinatenmessgerätes befestigt ist, ist ein Zusammensetzen mehrerer Computertomographie-Aufnahmen nicht möglich.

Darüber hinaus weisen Computer-Tomographen üblicherweise eine relativ grobe Messunsicherheit in der Größenordnung von 10 μm oder mehr auf. Die alleinige Messung des Messobjektes mit dem Computer-Tomographen - wie in der DE-A- 103 31 419 beschrieben - ist somit zur vollständigen Lösung der geometrischen Messaufgaben an üblichen Zeichnungsteilen nicht ausreichend. Ein weiteres Problem besteht darin, die geometrische Kalibrierung der Computer-Tomographen vorzunehmen. Da die Eigenschaften der tomographischen Messung stark von den Eigenschaften des Messobjektes selbst abhängen, ist dies nur schwer global an Messnormalen durchzuführen.

Aus der DE-A-100 44 169 ist ein Verfahren zur Bestimmung der Dicke von Werkstücken bekannt. Dabei trifft die ein zu messendes Bauteil durchsetzende Röntgenstrahlung auf einen Detektor. Das Bauteil kann mittels eines Manipulators gedreht sowie angehoben und abgesenkt werden. Nach vollständiger Durchleuchtung des Bauteils liefert ein Rechner eines Computer-Tomographen einen Stapel von Grauwertschnittbildem, die zusammengefügt werden, um einen dreidimensionalen Voxel-Datensatz zu erhalten. Aus diesem wird sodann die Wandstärke des Bauteils berechnet.

Aus der DE-C-38 06 686 ist ein Koordinatenmessgerät mit einem Multisensortastsystem bekannt, das einen taktil arbeitenden Sensor, einen Lasersensor und einen Videosensor umfasst, wobei entsprechend den Messaufgaben ein Einsatz einer der Sensoren erfolgt. Einen dieser Sensoren durch einen Computer-Tomographen zu ersetzen, sieht die EP-A-1 389 263 vor.

Bei der Verwendung einer Röntgensensorik sind umfangreiche Schutzmaßnahmen zur Abschirmung der Röntgenstrahlen erforderlich, um Strahlenschutzvorschriften zu genügen. Dabei muss sichergestellt sein, dass die Strahlenbelastung außerhalb der Messanordnung vorgegebene Grenzwerte nicht überschreitet. Um diesen Anforderungen zu genügen, ist es bekannt, dass um die Messanordnung, also unabhängig von dieser ein Strahlenschutzgehäuse angeordnet wird, das z. B. aus Blei oder Bleischichten aufweisendem Verbundmaterial besteht. Das Strahlenschutzgehäuse hat dabei die ausschließliche Aufgabe, die im Computer-Tomographen entstehende Röntgenstrahlung zu absorbieren. Durch das zusätzliche Gehäuse wird die Gesamtmessanordnung voluminös. Eine unerwünschte Gewichtserhöhung sowie hohe Kosten sind weitere Folgen.

Nachteil bekannter Computer-Tomographen ist es des Weiteren, dass die Messgeschwindigkeit hinter der zurückbleibt, die in der Koordinatenmesstechnik mit optischen Sensoren erzielbar ist. Nachteilig es auch, dass der Computer-Tomograph fest am Grundaufbau des Koordinatenmessgerätes befestigt sind, so dass die Mess-Einsatzmöglichkeit eingeschränkt ist.

Aus der US-A-2003/0043964 ist ein Inspektionssystem für Flugzeugrümpfe bekannt, das eine innerhalb des Rumpfes von einem Kran ausgehende Röntgenquelle und einen außerhalb des Rumpfes ebenfalls von einem Kran ausgehenden Sensor umfasst. Um die Position des Sensors zu bestimmen, wird ein Triangulationsverfahren benutzt.

Ein Messgerät nach der DE-A-100 01 239 sieht neben einem Positionsdetektor ein nicht optisches Messsystem wie ein AFM (Atomic Force Microscope) vor, die über ein Tragelement starr miteinander verbunden sind.

Ein Multisensormesskopf nach der DE-A-44 45 331 umfasst eine Vertikalachse, auf der mehrere Sensoren montiert sein können.

In einem Koordinatenmessgerät nach der EP-A-0 504 609 werden neben Messköpfen auch Gelenkfräsköpfe eingesetzt.

Eine Röntgenprüfanordnung nach der US-A-5,038,378 sieht die Möglichkeit vor, einen Röntgenstrahldetektor unabhängig voneinander entlang von drei Achsen zu verstellen.

Der vorliegenden Erfindung liegt die Aufgabe zu Grunde, ein Verfahren und ein Koordinatenmessgerät zum Messen eines Objektes mit zumindest einer Röntgensensorik als erste Sensorik derart weiterzubilden, dass auch Messobjekte größerer Ausdehnung problemlos gemessen werden können. Ferner soll im Vergleich zum Stand der Technik eine höhere Messsicherheit erzielbar sein. Des Weiteren soll auf einfache Weisung eine geometrische Kalibrierung der Röntgensensorik (Computer-Tomographen) ermöglicht werden. Die Vorrichtung soll kompakt aufgebaut sein, wobei gleichzeitig eine hinrechende Abschirmung gegen Röntgenstrahlung sichergestellt sein soll. Hohe Messdichten durch einfache Maßnahmen und hohe Messgeschwindigkeiten sollen realisierbar sein. Ferner soll eine Verbesserung der Auflösung sowie eine Reduzierung des Signal-/Rauschverhältnisses ermöglicht werden. Das Messen von Objekten, die in Bezug auf Röntgenstrahlung nur einen geringen Kontrast erzeugen, soll mit hinreichender Genauigkeit durchführbar sein.

Zur Lösung der Aufgabe sieht die Erfindung verfahrensmäßig u. a. vor, dass mehrere Ausschnitte des Messobjektes nacheinander durch die bekannten Verfahren der Tomographie erfasst werden und für die gesamte Menge der zusammengesetzten Durchstrahlungsbilder, die 3D-Rekonstruktion erfolgt, wobei vorzugsweise größere Messobjekte als durch das Sehfeld des Tomographen bedingt gemessen werden.

Auch zeichnet sich ein Koordinatenmessgerät der eingangs genannten Art dahingehend aus, dass die Röntgensensorik in dem Koordinatenmessgerät in x-, y- und/oder z-Richtung zu dem Objekt positionierbar ist und dass Drehachse des Objekts auf einer Verfahrachse zur Erweiterung des Messbereichs entlang der Drehachse angeordnet ist und dass Drehachse des Objekts auf einer Verfahrachse zur Erweiterung des Messbereichs entlang der Drehachse angeordnet ist.

Weiterbildungen ergeben sich aus den abhängigen Ansprüchen.

Zur Lösung eines Aspekts der Erfindung wird auch ein Koordinatenmessgerät zum Messen eines Objektes mit einer Röntgensensorik als erste Sensorik umfassend eine Röntgenstrahlenquelle und zumindest einen die Röntgenstrahlen erfassenden Röntgenstrahlensensor sowie - sofern vorhanden - eine zweite Sensorik wie taktile und/oder optische Sensorik die in x-, y- und/oder z-Richtung des Koordinatenmessgerätes relativ zu dem Objekt positionierbar ist, vorgeschlagen, das sich dadurch auszeichnet, dass die Röntgensensorik entsprechend der zweiten Sensorik in dem Koordinatenmessgerät positionierbar ist. Mit anderen Worten ist die Röntgensensorik gleichwertig zu der zweiten Sensorik im Koordinatenmessgerät angeordnet, wobei zum Positionieren der Röntgensensorik und Auswerten deren Messdaten prinzipiell die gleichen Komponenten bzw. Hard- und Software verwendet werden können, die auch für die weitere Sensorik vom Prinzip her zum Einsatz gelangen. Die zweite Sensorik selbst kann wiederum mehr als einen Sensor umfassen.

Erfindungsgemäß ist somit u. a. vorgesehen, dass die Röntgensensorik (Computer-Tomograph) nicht fest am Koordinatenmessgerät angebaut wird, sondern als Sensorik vollständig in das Koordinatenmessgerät integriert wird. Hierzu werden Sender und Empfänger des Computer-Tomographen so im Koordinatenmessgerät angeordnet, wie dies üblicherweise bei Durchlichtbeleuchtung und Bildverarbeitungssensor realisiert wird. Röntgenempfänger und - sofern vorhanden - Bildverarbeitungssensor oder auch mechanischer Taster können ist auf einer mechanischen Achse beweglich angeordnet. Gleichfalls ist es möglich für jeden Sensor separate Achsen einzusetzen. Die jeweiligen Strahlenquellen für Licht- und Röntgenstrahlung sind dem jeweiligen Sensor gegenüber angeordnet.

Durch den erfindungsgemäßen Aufbau ist es möglich, mehrere Ausschnitte des Messobjektes, zur hochgenauen Messung auch in hoher Auflösung, nacheinander durch die bekannten Verfahren der Tomographie (Drehen des Teiles und Aufnehmen von mehreren Durchstrahlungsbildern) zu erfassen. Anschließend kann für die gesamte Menge der zusammengesetzten Durchstrahlungsbilder die 3D-Konstruktion erfolgen. Es ist somit möglich größere Messobjekte als durch das Sehfeld des Tomographen bedingt zu messen.

Es werden erfindungsgemäß mehrere tomographische Bilder unter Einbeziehung des Koordinatenmessgerätes bzw. des Koordinatensystems des Messgerätes aneinander gereiht, die auch in unterschiedlichen Relativlagen zwischen Messobjekt und Röntgensensorik, insbesondere unterschiedlichen Vergrößerungen, aufgenommen werden, wobei die dabei erfassten Bereiche des Messobjektes sich nicht vollständig oder gar nicht überlappen. Bei der Zusammensetzung der Teilaufnahmen werden in den Überlappungsbereichen vorzugsweise die Messergebnisse, die in höherer Vergrößerung aufgenommen wurden, verwendet. Die Zuordnung der Ergebnisse in verschiedenen Vergrößerungen ist möglich, da die Vergrößerungseinstellung mit CNC-Achsen erfolgt und vorab eingemessen werden.

Darüber hinaus ist es möglich, genauer zu messende Merkmale des Messobjektes in traditioneller Weise mit den Sensoren des Multisensor-Koordinatenmessgerätes (z. B. taktil-optisch) zu messen. Röntgensensor und Bildverarbeitungssensor und taktiler Sensor messen wie bei Multisensor-Koordinatenmessgeräten üblich in einem gemeinsamen Koordinatensystem, sodass die Messergebnisse direkt zueinander in Bezug gesetzt werden können.

Bei dem gegebenen Aufbau ist es nun möglich, die Kalibrierung der Messungen mit der Röntgensensorik nach dem Tomographie-Prinzip direkt an den Messobjekten selbst durchzuführen. Hierdurch werden ausgezeichnete Punkte des Messobjektes mit der taktilen oder optischen Sensorik des Koordinatenmessgerätes in bekannter Genauigkeit gemessen. Diese Punkte werden bei der Auswertung der Berechnung der 3D-Rekonstruktion des Computer-Tomographen berücksichtigt, um die geometrische Kalibrierung dieses Rekonstruktionsprozesses durchzuführen.

Um bei kompaktem Aufbau eine hinreichende Abschirmung gegen Röntgenstrahlung sicherzustellen, sieht die Erfindung u. a. vor, dass die Abschirmung oder zumindest ein Bereich dieser als Funktionsbauteil für den erforderlichen messtechnischen Aufbau des Koordinatenmessgerätes ausgebildet ist. Hierbei kann es sich z. B. um Aufnahmen von mechanischer Achse oder diese selbst, Grundplatten, Lagerungen etc. handeln, ohne dass hierdurch eine Beschränkung der Erfindung erfolgt. Mit anderen Worten wird die für den Strahlenschutz benötigte Abschirmung ganz oder teilweise von Funktionsbauteilen des messtechnischen Aufbaus des Koordinatenmessgeräts übernommen.

Insbesondere besteht die Möglichkeit, dass Grundplatte und zumindest Rückwand des Gehäuses des Koordinatenmessgerätes derart dimensioniert bzw. aus einem Material hergestellt sind, dass gleichzeitig die erforderliche Abschirmung erzielt ist. Auch kann Grundplatte oder eine Seitenwandung entsprechend ausgebildet sein. Hierzu ist insbesondere vorgesehen, dass die für die Abschirmung relevanten Bauteile aus Hartgestein wie Granit bestehen. Aber auch andere Materialien, insbesondere Kunststeine, die ggf. mit einem entsprechenden strahlenabsorbierenden Material versetzt sind, können zum Einsatz gelangen.

In hervorzuhebender Weiterbildung der Erfindung ist vorgesehen, dass die Abschirmung bzw. diese bildende Bauteile des Koordinatenmessgerätes wie Wandung Montageort für Funktionskomponenten des Koordinatenmessgerätes sind. Folglich sind die abschirmenden Bauteile gleichzeitig zur Montage von Funktionskomponenten des Koordinatenmessgerätes, insbesondere des zum Einsatz gelangenden Computer-Tomographen verwendbar, wobei die Funktionskomponenten mechanische Achsen bzw. Verfahrachsen und/oder Sensoren und/oder Strahlen- bzw. Lichtquellen sein können.

Um eine hinreichende Abschirmung sicherzustellen, können die für die Abschirmung verwendeten Bauelemente stärker dimensioniert sein als dies aus messtechnischer oder statischer Sicht notwendig ist.

Nach einem eigenerfinderischen Vorschlag ist vorgesehen, dass der Röntgenstrahlenquelle mehrere Sensoren zugeordnet sind, deren das Objekt durchsetzende Bestrahlungswinkel voneinander abweichen, wobei insbesondere zum Messen des Objekts gleichzeitig mit Röntgenstrahlen beaufschlagte n-Sensoren der Röntgenstrahlenquelle zugeordnet sind, die Röntgenstrahlenquelle zwischen aufeinander folgenden Messungen relativ zum Objekt um einen Basiswinkel $\alpha$ verstellbar ist und aufeinander folgende Sensoren jeweils um einen Winkel $\alpha/n$ zum benachbarten Sensor verdreht angeordnet sind.

Die erfindungsgemäße Anordnung umfasst n Detektoren für die Röntgenstrahlung, die so angeordnet sind, dass mit jedem Detektor bzw. Sensor jeweils ein Durchstrahlungsbild mit einem unterschiedlichen Durchstrahlungswinkel aufgenommen wird, wodurch eine Verringerung der benötigten Winkelstellung zur Erzeugung eines Tomogramms notwendig ist.

Erfindungsgemäß werden die Röntgensensoren um eine Winkeldifferenz zueinander justiert, die sich wie folgt ergibt. Es wird ein Basiswinkel verwendet, der sich als ganzteiliges Vielfaches des verwendeten Winkelschritts zwischen Strahlungsquelle und Sensor einerseits und zu messendem Objekt andererseits ergibt, wobei insbesondere das Objekt sich auf einem Drehtisch befindet, der zu der Röntgenstrahlensensorik drehbar ist. Der Winkel des zweiten Sensors wird um einen Wert 1/(Anzahl der Sensoren) erhöht, der Winkel des dritten Sensors wird um einen Wert 2/ (Anzahl der Sensoren) erhöht. Folglich wird der n-te Sensor um (n-1) / (Anzahl der Sensoren) erhöht. Somit ist die Möglichkeit gegeben, bei einmaliger Umdrehung die u-fache Anzahl von Winkelstellungen auszuwerten, wobei u = das Produkt aus n = Anzahl der Sensoren und m = Anzahl der Positionierungen des Objekts zu der Röntgensensorik ist.

Erfmdungsgemäß werden die Röntgensensoren um ganzzahlige Vielfache des Winkelschritts des Drehtischs zueinander justiert, wobei die Durchstrahlungszeit in der jeweiligen Winkelposition verkürzt werden kann. Ungeachtet dessen wird die mehrfache Menge von Röntgenstrahlen durch die mehrfache Anzahl von Sensoren aufgenommen, wodurch das Signal-Rauschverhältnis reduziert wird.

In eigenerfinderischer Weiterbildung ist vorgesehen, dass bei der Bildaufnahme bzw. Bildübertragung oder Bildauswertung jeweils mehrere Pixelelemente des Sensors zu einem Pixel zusammengefasst werden und die ursprüngliche Auflösung in dem Volumenbild, das aus den Bildern mit entsprechend reduzierter Pixelzahl berechnet wurde, durch rechnerische Interpolation erreicht bzw. übertroffen wird.

So kann nach Aufnahme der 2D-Bilder mittels der Tomographie das vorhandene 2D-Bild z. B. durch Mittelwertbildung aus benachbarten Pixeln in ein geringer aufgelöstes Bild mit weniger Pixelinformation umgerechnet werden. Aus diesen Bildern geringer Auflösung erfolgt sodann eine 3D-Rekonstruktion zur Errechnung eines dreidimensionalen Voxel-Bildes aus den verschiedenen 2-D-Durchstrahlungsbildern. Nach Bestimmung dieses Voxel-Bildes wird das Voxel-Bild in ein Bild der ursprünglichen Auflösung durch Interpolation zwischen mehreren Voxeln zurückgerechnet. Dabei ist es sogar möglich, durch weitere Anwendung der gleichen Vorgehensweise unter Einsatz bestimmter Algorithmen zusätzliche Voxel zu berechnen und somit eine höhere Auflösung des Voxel-Bildes zu erreichen.

Eigenerfinderisch ist auch der Gedanke, dass das Objekt während der Messung kontinuierlich gedreht wird, wobei die Röntgenstrahlenquelle mit Hilfe z. B. eines mechanischen oder elektronischen Verschlusses oder gleichwirkenden Maßnahmen wie hochfrequente Modulierung nur mehrfach kurzzeitig geöffnet wird, um eine Bewegungsunschärfe zu vermeiden. Ungeachtet dessen wird auf diese Weise eine Messzeitverkürzung erzielt.

In Weiterbildung ist auch vorgesehen, dass von dem Objekt mehrere Tomogramme unter Ausnutzung verschiedener Spektralbereiche der Röntgenstrahlung erzeugt werden können. So wird der Spektralbereich der Röntgenstrahlung durch die Kathodenspannung des Röntgenstrahlers bestimmt. Typisch - jedoch rein beispielhaft - kann z. B.

sein, dass das Messobjekt mit einer Kathodenspannung von 50 kV und 90 kV und 130 kV tomographiert wird, um sodann aus den Unterschieden der Messergebnisse bei den verschiedenen Kathodenspannungen, d. h. Röntgenlichtfarbe bzw. Röntgenstrahlungsfrequenz auf die Größe von systematischen Messabweichungen wie z. B. Artefakten zu schließen und diese sodann zu korrigieren.

Es können auch mehrere Tomogramme vom Objekt aufgenommen werden, wobei der Winkel zwischen Drehachse des das Objekt aufnehmenden Drehtisches und Röntgenstrahlenquelle und zugeordneten Sensoren mit Hilfe mechanischer Dreh-Schwenkachsen oder durch den Einsatz mehrerer Detektoren unter verschiedenen Winkeln variiert werden kann, wobei sich die Sensoren insbesondere entlang einer Geraden erstrecken, die parallel zur Drehachse des Drehtisches verläuft.

Zur Erhöhung der Auflösung im Tomogramm können mehrere Aufnahmen aufgenommen werden, zwischen denen der Sensor bzw. das Objekt um einen Abstand verschoben wird, der kleiner als Kantenlänge eines empfindlichen Elements des Sensors ist.

Des Weiteren sieht die Erfindung vor, dass das Objekt von parallel zueinander verlaufenden Röntgenstrahlen durchsetzt wird. Hierzu wird die Röntgenstrahlung mit Hilfe geeigneter Einrichtungen parallelisiert.

Ferner bzw. alternativ besteht die Möglichkeit, dass mit Hilfe translatorischer Relativbewegungen zwischen dem zu messenden Objekt und Röntgenstrahlenquelle/Sensor ein Bereich aufgenommen wird, der größer als Fläche des Sensors ist.

Um auch Werkstücke aufzunehmen, die in Bezug auf Röntgenstrahlung nur einen geringen Kontrast zeigen, sieht die Erfindung vor, dass das Objekt von einem Material umgeben ist, das eine höhere Absorption als das Objekt selbst aufweist. So kann bei einem Messobjekt z. B. aus einem Stoff mit geringer Massenzahl wie z. B. Lithium der Kontrast der tomographisch ermittelten 2-D-Durchstrahlungsbilder dadurch verbessert werden, dass ein Ausgießen des Messobjekts mit einem schwereren Material erfolgt. Somit werden von der Negativform des Messobjekts hinreichend kontrastreiche Bilder gewonnen, die wiederum eine Darstellung des Messobjekts ermöglichen.

Um eine Messoptimierung zu erreichen, kann mit verschiedenen Sensoren gemessen werden. Hierzu ist vorgesehen, dass neben der Röntgenstrahlenquelle und den diesen zugeordneten Sensoren weitere Sensoren für die messtechnische Erfassung des Objekts wie z. B. mechanische Taster, Lasertaster, Bildverarbeitungssensoren in der Anordnung vorgesehen sind, die gegebenenfalls auf separaten Verfahrachsen angeordnet sind.

Auch kann die zur Aufnahme eines Tomogramms benötigte Drehachse für die Drehung des Objekts auf einer Verfahrachse angeordnet sein, wodurch der Messbereich in Richtung der Drehachse erweitert wird. Mit anderen Worten kann das Objekt in Richtung der Drehachse verstellt werden. Dazu kann die Verfahrachse auch auf der Drehachse angeordnet sein.

Ein eigenerfinderischer Vorschlag zum Kalibrieren der Röntgensensorik in dem Koordinatenmessgerät sieht vor, dass ausgezeichnete Punkte des zu messenden Objekts mit taktiler und/oder optischer Sensorik gemessen werden und hieraus Geometriemerkmale wie Durchmesser oder Abstände ermittelt werden, die nach Bestimmung der gleichen Geometriemerkmale mit der Röntgensensorik zur Kalibrierung der Röntgensensorik herangezogen werden.

Somit können die durch die taktile und/oder optische Sensorik ermittelten Messergebnisse zu ausgezeichneten Punkten wie Randbereiche des Messvolumens werden zur Korrektur der über Schwellwertverfahren aus den 3D-Voxel-Daten generierten Messpunktwolke herangezogen werden.

Die nach dem Tomographieren eingesetzte Schwellwertoperation führt zur Gewinnung von 3D-Punktewolken, die im ASCI-Format oder STL-Format dargestellt sein können. Diese Punktewolke wird zwischen dem taktil oder optisch ermittelten Messpunkt so korrigiert, dass die Abweichungen zwischen taktiler und/oder optischer Messung und der tomographischen Messung ein Minimum werden. Zwischen den taktilen und/oder optischen Messpunkten wird hierbei eine Interpolation zur Bestimmung der Abweichung vorgenommen.

Bei der Berechnung der Voxel mittels 3D-Rekontruktion werden die Positionen der Voxel um den mit taktiler bzw. optischer Gegenmessung ermittelten Korrekturwert verändert, die sich an den Materialgrenzen (Ränder des Messvolumens) des tomographierten Objekts befinden. Zwischen Stützpunkten liegende Voxel-Positionen werden anschließend durch Interpolation zwischen den gemessenen Korrekturpositionen korrigiert. Hierdurch entsteht ein nicht regelmäßiges Voxel-Raster im dreidimensionalen Raum, wobei die Voxel-Orte besser der wirklichen Objektgeometrie als dem Original Voxel-Bild entsprechen. Vorteilhafterweise wird dieses Voxel-Bild sodann in ein regelmäßiges Raster resampelt. Dies kann dadurch geschehen, dass ein Sollraster für das Voxel-Bild vorgegeben wird und für jeden Punkt des Sollrasters aus den in der Umgebung befindlichen Voxel-Amplituden eine neue Voxel-Amplitude durch Interpolation errechnet wird.

Um eine hohe Messsicherheit zu erzielen und auf einfache Weise eine geometrische Kalibrierung der Röntgensensorik (Computer-Tomographen) zu ermöglichen, schlägt die Erfindung vor, dass die Position für Röntgenquelle und Röntgendetektor nach einem einmaligen Einmessen für bestimmte Vergrößerungs- und Messereichsanordnungen mit den zugehörigen Kalibrierdaten gespeichert werden und dann softwaregesteuert beliebig ohne weitere Nachkalibrierung für nachfolgende Messungen abrufbar sind.

Mit anderen Worten ist erfindungsgemäß vorgesehen, dass alle Einstellparameter, die für das Tomographieren mit einer bestimmten Vergrößerung oder einem bestimmten Messbereich notwendig sind - hierzu gehört die Position der verschiedenen Achsen des Tomographie- bzw. Koordinatenmessgerätes -, sowie die diesen Positionen zugeordneten Vergrößerungswerte und andere Kalibrierdaten inklusive Korrekturwerte zur Position der Achsen bezüglich des durch die zugeordneten Wegmesssysteme gelieferten Positionsmesswertes in einem einmaligen Einmessvorgang des Koordinatenmessgerätes messtechnisch erfasst und abgespeichert werden. Diese abgespeicherten Werte werden sodann bei normalem Betrieb des Koordinatenmessgerätes durch Knopfdruck des Bedieners bzw. durch Aufruf aus einem CNC-Programm abgerufen, die Maschinen in die entsprechenden Positionen verfahren und die zugehörigen Kalibrierdaten beim nachfolgenden Messprozess benutzt.

Insbesondere ist vorgesehen, dass vorab eingemessene Vergrößerungs- und Messbereichseinstellungen durch das Messprogramm des Koordinatenmessgerätes automatisch aufgerufen und die entsprechenden Hardware-Komponenten des Gerätes positioniert werden.

Ferner besteht die Möglichkeit, dass Röntgenquelle und Röntgendetektor synchron verfahren werden, um lediglich die Vergrößerung und/oder Messbereich zu verändern bzw. dass Röntgenquelle und Röntgendetektor unabhängig voneinander verfahren werden, um Vergrößerung und/oder Messbereich zu verändern. Messergebnisse aus verschiedenen Vergrößerungen können dadurch kombiniert werden.

Es besteht auch die Möglichkeit, dass alle für das Röntgenmessen (Tomographieren) notwendigen Einstellungen im Vorhinein eingemessen und abgespeichert werden und beim jeweiligen Röntgenmessvorgang wie Tomographiervorgang keinerlei Einmessvorgänge mehr notwendig sind.

Die Drehmittelpunktjustage kann durch einen Einmessvorgang und/oder eine entsprechende Korrektur des Drehmittelpunktversatzes in der Software realisiert werden.

Eine Weiterbildung sieht vor, dass die Bestimmung der Vergrößerung für die Tomographie und/oder der Drehmittelpunktlage in Bezug auf Röntgenquelle und Röntgendetektor mittels eines Normals bestimmt wird, das aus mindestens zwei Kugeln besteht. Insbesondere ist vorgesehen, dass das Normal aus vier Kugeln besteht.

Ein Verfahren zur Bestimmung der Position des Drehmittelpunkts im Koordinatenmessgerät zeichnet sich insbesondere durch die Verfahrensschritte aus

- ein Vierkugelnormal bestehend aus vier in den Ecken eines Rechtecks wie Quadrats angeordneten Kugeln, bei dem die Abstände der Kugeln zueinander bekannt bzw. kalibriert sind, wird auf der Drehachse positioniert,
- das Vierkugelnormal wird so verdreht, dass die aufgespannte Ebene parallel zum Detektor steht,
- Messung der Vierkugelposition im Messfeld des Detektors,
- Berechnung der mittleren Vergrößerung M1 aus den vier gemessenen Kugeldistanzen, den nominalen Kugeldistanzen und der nominalen Pixelgröße des Detektors,
- Drehung der Drehachse um 180°,
- Messung der vier Kugelpositionen im Bild,
- Berechnung der mittleren Vergrößerung M2 aus den vier gemessenen Kugeldistanzen, den nominalen Kugeldistanzen und der nominalen Pixelgröße des Detektors.

Die Berechnung der Y-Position des Drehmittelpunktes anhand der vier Kugelpositionen vor und nach der Drehung erfolgt nach folgender Gleichung: Pdyn = (Pkyn1 * M2 + Pkyn2 * M1) /(M1 + M2) mit Pdyn: Y-Position der Drehachse auf dem Detektor für Kugel n, Pkyl : Y-Position der Kugel n ei Drehwinkel 0°, Pkyn2: Y-Position der Kugel n bei Drehwinkel 180°, M1: mittlere Vergrößerung bei Drehwinkel 0°, M2: mittlere Vergrößerung bei Drehwinkel 180°.

Es besteht auch die Möglichkeit, dass das Messen eines Objektes mit einem Koordinatenmessgerät erfolgt, das neben der Röntgensensorik (Computer-Tomographen) weitere Sensoren aufweist, so dass Messungen mittels einer taktilen und/oder optischen Sensorik durchgeführt werden können, wobei insbesondere taktil-optische Messungen zu erwähnen sind. Somit besteht die Möglichkeit, dass eine Korrektur der mit der Röntgensensorik bzw. Tomographie gemessenen Messpunktewolke des Messobjektes oder der hieraus errechneten triangulierten Flächenelemente durch taktil oder optisch gewonnene Messpunkte erfolgt, wobei zwischen den taktil und/oder optisch gemessenen Korrekturpunkten interpoliert werden kann.

Auch kann zwischen den mit taktiler und/oder optischer Sensorik gewonnenen Korrekturpunkten unter Berücksichtigung des Funktionsverlaufs der durch Röntgenmessung wie Tomographie gewonnenen Punktewolke interpoliert werden und/oder durch Berücksichtigung des Nominal-CAD-Models interpoliert werden.

Ein weiterer Vorschlag sieht vor, dass zuerst ein Musterteil des Messobjekttyps mittels Röntgenstrahlen (tomographisch) und taktil und/oder optisch abgetastet wird, aus der Differenz beider Messungen ein Korrekturnetzwerk für die Korrektur der tomographischen Messwerte errechnet wird und beim Messen von Serienteilen die tomographischen Messungen mit den einmalig bestimmten Korrekturwerten korrigiert werden.

Die diesbezügliche Vorgehensweise ist so zu verstehen, dass in einem ersten Messvorgang viele Messpunkte zu einem typischen Vertreter des Messobjektes mittels Röntgenstrahlen tomographisch und taktil oder optisch gemessen werden. Hierbei wird auch beim taktilen und/oder optischen Messen mit sehr vielen Messpunkten gearbeitet, um ein ausreichend dichtes Korrekturnetzwerk zu erzielen. Hieraus werden sodann für jeden Oberflächenort des zu tomographierenden Objektes entsprechende Korrekturwerte ermittelt, die sich aus dem Vergleich der taktilen und/oder optischen Messwerte mit den tomographischen Messwerten ergeben. Beim späteren Tomographieren von weiteren Teilen werden diese Korrekturwerte direkt zum Ansatz gebracht. Ein nochmaliges taktiles oder optisches Gegenmessen ist nicht erforderlich.

Folglich ist vorgesehen, dass ein kalibriertes Teil eines Messobjektes tomographiert wird und aus der Messabweichung bei der Messung ein Korrekturnetzwerk für die Korrektur der tomographischen Messwerte errechnet wird und beim Messen von Serienteilen die tomographischen Messungen mit den vorher abgestimmten Korrekturwerten abgestimmt werden.

Bei der Messung von Serienteilen können zusätzlich einzelne optisch und/oder taktil gemessene Korrekturpunkte berücksichtigt werden.

Die taktilen und/oder optischen Messpunkte können zur Korrektur durch einen Bediener grafisch an der durch Tomographie bestimmten Punktwolke festgelegt werden und durch das Koordinatenmessgerät dann automatisch gemessen werden.

Eine Weiterbildung sieht vor, dass die taktilen und/oder optischen Messpunkte zur Korrektur durch einen Bediener grafisch an dem dem zu messenden Teil zugrunde liegenden CAD-Modell festgelegt werden und durch das Koordinatenmessgerät dann automatisch gemessen werden. Dabei können die taktilen und/oder optischen Messpunkte zur Korrektur durch einen automatischen Algorithmus auf der Oberfläche des dem zu messenden Teil zugrunde liegenden CAD-Modells annähernd gleichmäßig oder gleichmäßig verteilt durch das Koordinatenmessgerät automatisch gemessen werden. Auch können die taktilen und/oder optischen Messpunkte zur Korrektur durch einen Bediener grafisch am CAD-Modell festgelegt und nach dem Laden des CAD-Modells durch das Koordinatenmessgerät automatisch gemessen werden.

Die Erfindung lehrt des Weiteren, dass beim Tomographiervorgang grundsätzlich ein Kalibrierkörper, insbesondere eine Anordnung von Kugeln mit tomographiert wird und hieraus die Relativlage der Drehachse zum Koordinatenmessgerät und/oder zur Röntgenquelle und/oder zum Röntgensensor bestimmt und anschließend mathematisch korrigiert wird. Dabei kann die Lage der Kalibrierkörper auf der Drehachse mit optischen und/oder taktilen Sensoren bestimmt und zur Korrektur der Lage der Drehachse herangezogen werden.

Insbesondere ist jedoch vorgesehen, dass der Kalibrierkörper wie die zumindest zwei Kalibrierkugeln in der Halterung, d. h. dem Drehtisch des zu messenden Objektes in einem Material eingelagert sind, das eine geringe Absorption gegenüber Röntgenstrahlung aufweist. Somit kann durch die Erfassung des Kalibrierkörpers das Messobjekt auf dem Drehtisch positioniert werden, da mittels des Kalibrierkörpers die Drehpunktlage, also die Drehachse des Drehtisches ermittelt werden kann.

Die Raumlage der Rotationsachse in Bezug zur Röntgenquelle und Röntgendetektor kann erfindungsgemäß mit der Röntgensensorik und/oder mit der taktilen Sensorik und/oder mit der optischen Sensorik messtechnisch bestimmt und diese Lageabweichung beim Tomographieren von Messobjekten mathematisch korrigiert werden.

Insbesondere wird die von der Nominallage abweichende Lage der Drehachse durch Rotation und/oder Translation und/oder Verzerrung der 2D-Einzelbilder korrigiert.

Des Weiteren kann die von der Nominallage abweichende Lage der Drehachse im Rekonstruktionsalgorithmus berücksichtigt werden.

Ein weiterer Vorschlag der Erfindung sieht vor, dass das Messobjekt durch Einsatz von taktilen und/oder optischen Sensoren und/oder Tomographie in seiner Lage auf dem Drehtisch des Messgerätes und somit im Maschinenkoordinatensystem bestimmt wird und anschließend im 2D-Durchstrahlungsmodus an eingemessener Position des Röntgensensors durch Maße mit Methoden der Bildverarbeitung gemessen werden.

Durch diese Maßnahmen wird ein weiteres Nutzungsprinzip des erfindungsgemäßen Koordinatenmessgerätes, in dem eine Röntgensensorik integriert ist, verdeutlicht. So wird die Möglichkeit geschaffen, in 2D-Durchstrahlungsbildem zu messen. Üblicherweise ist dies nicht möglich, da die aktuelle Vergrößerung am Messobjekt nicht bekannt ist; denn die Position des Messobjekts im Röntgenstrahlengang ist unbekannt, die Position jedoch die Vergrößerung nach dem Strahlensatz wesentlich bestimmt. Wird nun die Position des Messobjekts im Koordinatenmessgerät mit einer optischen und/oder taktilen Sensorik genau bestimmt, ist aus dieser Position heraus die Vergrößerung des aktuellen durchstrahlten Messobjekts bekannt und der Einsatz der Röntgensensorik zur zweidimensionalen Messung mit Methoden der Bildverarbeitung möglich.

Der Röntgendetektor kann automatisch durch die Geräte-Software so gesteuert werden, dass der Detektor während dem eigentlichen Tomographievorgang in den Strahlkegel der Röntgenquelle positioniert und außerhalb dieser Zeiten außerhalb des Strahlungskegels in Parkstellung gebracht wird.

Durch diese Maßnahmen wird die Bestrahlungsbelastung des Röntgensensors minimiert und somit dessen Lebensdauer verlängert.

Eigenerfinderisch ist vorgesehen, dass Bildverarbeitungssensorik und Röntgensensorik eines Multisensorkoordinatenmessgerätes mit der gleichen Bildverarbeitungs-Hardware und der gleichen Bildverarbeitungs-Software bzw.

Teilen davon ausgestattet sind. Dabei sind die von der Bildverarbeitungssensorik bekannten Bildbearbeitungsverfahren ebenfalls für die Röntgensensorik anwendbar.

Des Weiteren sieht die Erfindung vor, dass die 2D-Röntgenbilder vor Rekonstruktion einer Verzeichnungskorrektor und/oder einer Hellsignalkorrektur und/oder einer Dunkelsignalkorrektur und/oder einer mathematischen Translation und/oder einer mathematische Rotation und/oder einem Resamplingverfahren und/oder einer Linearitätskennlinienkorrektur und/oder einer Bildverarbeitungsfilterung unterzogen werden.

Bevorzugte Weiterbildungen der Erfindung sind sowohl den unabhängigen als auch den abhängigen Ansprüchen zu entnehmen.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung von der Zeichnung zu entnehmenden bevorzugten Ausführungsbeispielen.

Es zeigen:

Fig. 1     eine Prinzipdarstellung eines Multisensorkoordinatenmessgerätes,

Fig. 2     ein Funktionsprinzip eines 3D-Computer-Tomographen,

Fig. 3     eine weitere Prinzipdarstellung eines Koordinatenmessgerätes,

Fig.4     eine Prinzipdarstellung einer ersten Anordnung von Röntgenstrahlenquelle und zugeordneten Sensoren,

Fig. 5     eine Prinzipdarstellung einer zweiten Anordnung einer Röntgenstrahlenquelle und zugeordnete Sensoren,

Fig. 6.     eine Prinzipdarstellung zur Bildauswertung,

Fig. 7     eine Prinzipdarstellung zur Erläuterung eines Verfahrens zur Erhöhung der Auflösung eines Tomogramms,

Fig. 8     eine Prinzipdarstellung eines Kalibrierkörpers,

Fig. 9     eine Prinzipdarstellung eines Drehtisches mit einem Kalibrierkörper,

Fig. 10     ein Blockschaltbild und

Fig. 11     Prinzipdarstellungen zur Erläuterung eines Korrekturverfahrens.

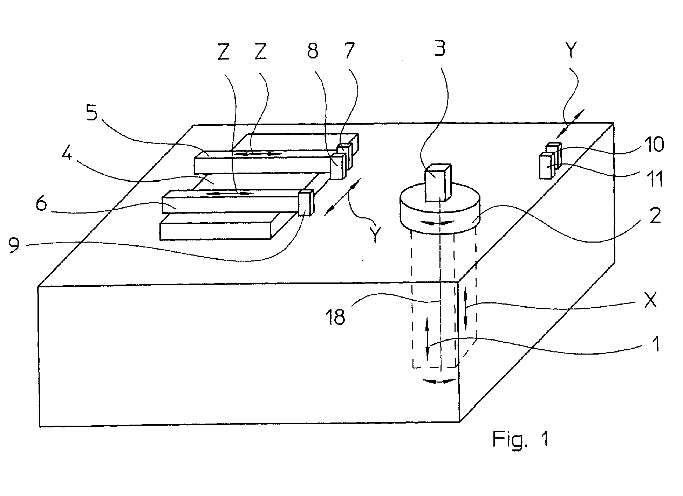

In der Fig. 1 ist prinzipiell ein Koordinatenmessgerät für den kombinierten Einsatz von Röntgensensorik und optischer und taktiler Sensorik dargestellt, gleichwenn die erfindungsgemäße Lehre in Wesentlichen Merkmalen auch für ein Koordinatenmessgerät geeignet ist, das neben dem Computer-Tomographen keine zusätzliche Sensorik umfasst.

Auf einer parallel zur X-Achse des Koordinatenmessgeräts verlaufenden Achse 18 ist ein Drehtisch 2 angeordnet. Auf diesem befindet sich ein Messobjekt 3 und kann somit um die Drehachse 18 rotiert werden und in X-Richtung durch die Achse 18 verschoben werden (Doppelpfeil). Auf einem parallel zu Y-Achse verlaufenden Schieber 4 sind zwei parallel zur Z-Achse verlaufende Achsen 5, 6 angeordnet. Auf der mechanischen Achse 5 befindet sich ein Sensor 7 für Röntgenstrahlung und ein Bildverarbeitungssensor 8. Auf der mechanischen Achse 6 befindet sich zusätzlich ein taktiler Sensor 9. Gegenüber dem Röntgensensor 7 ist eine Röntgenquelle 10 angeordnet, die wahlweise in Y-Richtung bewegbar oder fest angebracht sein kann. Gegenüber der Bildverarbeitungssensorik 8 befindet sich eine Durchlichtquelle 11. Die mechanischen Achsen bzw. Schieber, die entlang der X-, Y- bzw. Z-Achse des Koordinatenmessgerätes verlaufen, sind so ausgelegt, dass die in bzw. auf dem Koordinatenmessgerät installierten Sensoren jeweils den gesamten Messbereich auf dem Drehtisch 2 überdecken können.

Durch die Integration der Computer-Tomographie (CT) in ein Multisensor-Koordinatenmessgerät werden völlig neue Möglichkeiten geschaffen. Eine schnelle, zerstörungsfreie Komplettmessung mit der Tomographie wird mit hochgenauen Messungen von Funktionsmaßen mit taktiler oder optischer Sensorik vereint. Dabei ist erfindungsgemäß vorgesehen, dass die Röntgensensorik (Sensor, Strahlenquelle) entsprechend der zweiten Sensorik (z. B. Bildverarbeitungssensor, Durch- oder Auflichtstrahlenquelle oder taktiler Sensor gegebenenfalls mit zugeordnetem Bildverarbeitungssensor) in dem Koordinatenmessgerät positionierbar ist, dass also gleichwertig zur zweiten Sensorik die Röntgensensorik angeordnet ist. Dabei kann die Röntgensensorik mit zumindest der taktilen Sensorik und/oder der optischen Sensorik auf einer gemeinsamen mechanischen Achse angeordnet bzw. auf einer separaten mechanischen Achse angeordnet sein, die in analoger Weise wie die mechanischen Achsen für die taktile und/oder optische Sensorik arbeitet.

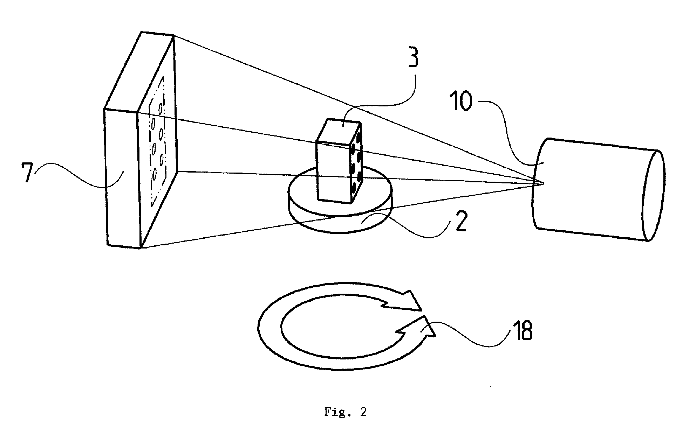

Das Funktionsprinzip der 3D-Computer-Tomographie soll anhand der Fig. 2 noch einmal erläutert werden.

Dabei werden für der Fig. 1 zu entnehmenden Elemente gleiche Bezugszeichen verwendet.

Das Werkstück 3 wird auf einem Drehtisch 2 aufgelegt und mit Röntgenstrahlen durchleuchtet. Der Sensor 7 z. B. in Form eines Flächendetektors wandelt das Röntgenbild in ein digitales 2D-Bild zur weiteren Verarbeitung um. Das Objekt 3 wird um 360° gedreht und Röntgenbilder in mehreren Drehlagen aufgenommen. Anschließend erfolgt auf der Basis der 2D-Bilder eine 3D-Rekonstruktion von Messpunkten, die die gesamte zu messende Werkstückgeometrie beschreibt. Sofern gewünscht kann durch Integration eines oder mehrerer der weiteren Sensoren 8, 9 der Einsatzbereich des Computer-Tomographen erweitert werden. Mit dem Bildverarbeitungssensor 8 ist die vollautomatische Messung von komplizierten, extrem kontrastarmen Werkstücken im Durch- und Auflicht möglich. Berührende Tastsysteme ermöglichen hochgenaue Messungen von optisch nicht zugänglichen Merkmalen.

Es besteht die Möglichkeit, dass der Sensor 7 und die Röntgenstrahlenquelle 10 synchron, also bei gleich bleibendem Abstand zueinander zu dem Objekt 3 verstellt werden. Hierdurch besteht die Möglichkeit einer Messbereichsanpassung, wobei diese gegebenenfalls automatisch erfolgen kann. Alternativ kann auch das Objekt 3 zu dem Sensor 7 verstellt werden, um so eine Anpassung an die Werkstückgröße und an die Genauigkeitsanforderungen zu ermöglichen. Wird das Objekt 3 zum Sensor 7 hin verstellt, so ergibt sich eine niedrigere Vergrößerung, wohingegen beim Verstellen des Objektes 3 zu der Röntgenstrahlenquelle 10 hin eine hohe Vergrößerung erzielbar ist. Auch kann der Sensor bei stationärer Röntgenstrahlenquelle 10 zu dem Objekt 3 verstellt werden.

Die erfindungsgemäße Lehre bietet insbesondere nachstehende Vorteile:

- Vollständige Erfassung aller Regel- und Freiformgeometrien am Werkstück in einem Messvorgang,
- Messen von Innengeometrien und nicht zugänglichen Merkmalen (z.B. verdeckte Kanten, Hinterschneidungen),
- 2D- und 3D-Messung von Form, Maß und Lage,
- umfangreiche Funktionen zur 2D-Messung im Röntgenbild,
- 3D-Soll-Ist-Vergleich als 3D-Abweichungsdarstellung im Vergleich zum 3D-CAD-Modell,
- Generierung von 3D-CAD-Daten aus gewonnenen CT-Daten,
- Kombination von Messergebnissen aus verschiedenen Vergrößerungen, und sofern mehrere Sesonsoren integriert sind:

- hochgenaue Messung von Funktionsmaßen mit taktiler oder optischer Sensorik,
- Rückführung der tomographischen Messergebnisse durch Multisensorik,
- kombiniertes Messen mit Tomographie und anderen Sensoren in einem Messablauf.

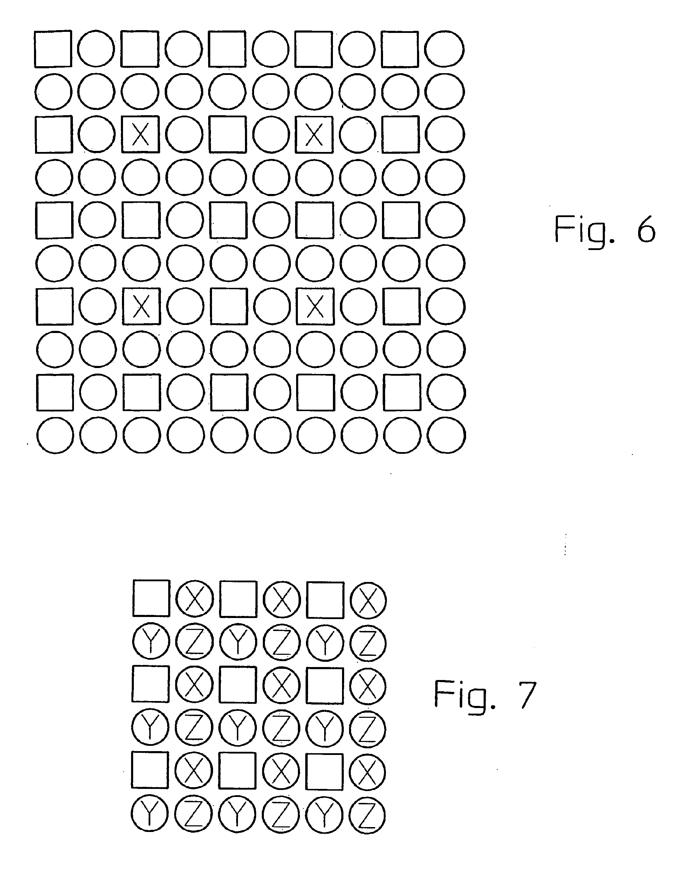

Anhand der Fig. 6 sei ein weiteres die Erfindung kennzeichnendes Verfahren erläutert, mittels dem eine Datenkompression möglich ist, ohne bezüglich der Auflösung Nachteile in Kauf nehmen zu müssen. Vielmehr besteht aufgrund der entsprechenden Lehre sogar die Möglichkeit, die Ursprungsauflösung zu übertreffen. Dies sei anhand eines 2D-Bildes erläutert.

In Fig. 6 stellen die Quadrate die Pixel eines 2D-Bildes dar. Das vorhandene 2D-Bild wird z. B. durch Mittelwertbildung aus benachbarten Pixeln in ein geringer aufgelöstes Bild mit weniger Pixelinformation (Pixel als Kreuze dargestellt) umgerechnet. Aus entsprechenden 2D-Durchstrahlungsbildern geringer Auflösung erfolgt sodann eine 3D-Rekonstruktion zur Berechnung des dreidimensionalen Voxel-Bildes. Nach Bestimmung dieses Voxel-Bildes wird das Voxel-Bild, das in der der Fig. 6 zu entnehmenden 2D-Darstellung ebenfalls durch Kreuze simuliert ist, in ein Bild der ursprünglichen Auflösung durch Interpolation zwischen mehreren Voxel-Bilden zurückgerechnet, so dass sich - ebenfalls in 2D-Darstellung - wieder ein Bild mit Quadraten ergibt. Darüber hinaus besteht die Möglichkeit, durch weitere Anwendung der gleichen Vorgehensweise zusätzliche Voxel zu berechnen, um eine höhere Auflösung des Voxel-Bildes zu erreichen. Dies wird durch die Kreise symbolisiert.

Auf diese Weise kann schneller gerechnet werden, da eine geringere Auflösung zunächst zu Grunde gelegt werden kann, ohne im Endeffekt Einbußen in der Auflösung hinnehmen zu müssen, diese sogar übertreffen kann.

Unter Bezugnahme auf die Fig. 7 soll ein weiteres erfindungsgemäßes Verfahren veranschaulicht werden, durch das die Auflösung im Tomogramm erhöht werden kann. Hierzu werden mehrere Aufnahmen aufgenommen, zwischen denen der Sensor zu dem Objekt bzw. das Objekt zu dem Sensor um eine Strecke verschoben wird, die kleiner als Kantenlänge eines empfindlichen Elements des Sensors ist. So ist in Fig. 7 der verwendete Röntgendetektor (Sensor) in seiner Auflösung durch die als Quadrate gezeichneten Pixel gekennzeichnet. Beim Tomographieren wird in jeder Drehstellung sowohl ein Bild in der als Quadrate gezeichneten Position des Röntgendetektors aufgenommen, als auch als Bild in der als Kreise mit X gekennzeichneten Position des Röntgendetektors als auch in der als Kreise mit Y gekennzeichneten Position des Röntgendetektors als auch in der als Kreise mit Z gekennzeichneten Position des Röntgendetektors aufgenommen. Alle Bilder werden zu einem Bild zusammengefügt und als Ganzes beim Tomographie-Rekonstruktionsprozess berücksichtigt. Es wird somit eine höhere Auflösung erreicht, als durch den Detektor physikalisch gegeben ist.

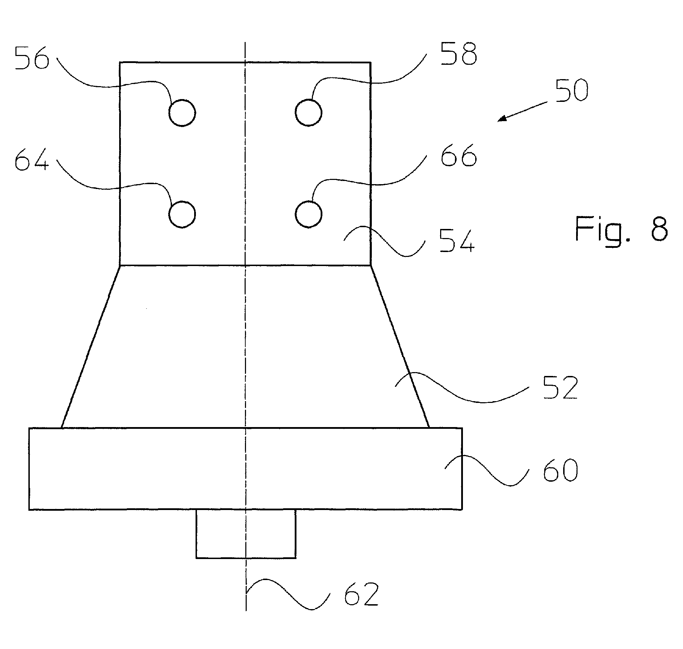

Um die Vergrößerung für die Tomographie und/oder den Drehmittelpunkt des Drehtisches 2 in Fig. 1 in Bezug auf die Röntgenquelle 10 bzw. den Sensor 7 zu bestimmen, kann ein Normal benutzt werden, das im Ausführungsbeispiel der Fig. 8 mit 50 gekennzeichnet ist. In der Prinzipdarstellung geht von einem Standfuß 52 ein Träger 54 aus einem Material aus, das eine geringe Absorption gegenüber Röntgenstrahlen aufweist. In dem Träger 54 sind zumindest zwei Kugeln 56, 58 aus Röntgenstrahlen stark absorbierendem Material wie Stahl angeordnet. Das Normal 50 wird sodann auf einen Drehtisch 60, der dem Drehtisch 2 der Fig. 1 entspricht, eines Tomographen angeordnet. Der Drehtisch 60 ist um eine Achse 62 drehbar, die mit der X-Achse des Koordinatenmessgerätes zusammenfallen kann. Die Einmessvorgänge zur Bestimmung der Lage der Drehachse 62 des Tomographen innerhalb des Koordinatenmessgerätes werden nun durch Messungen der Lagen der Kugeln 56, 58 relativ zum Röntgensensor 7 in verschiedenen Drehlagen des Kugelnormals 50 bestimmt.

Soll die Vergrößerung ermittelt werden, so sind Messungen in zwei verschiedenen Abständen zu dem Sensor 7 notwendig.

Um eine höhere Genauigkeit zu erzielen, kann das Normal 50 zwei weitere Kugeln 64, 66 aufweisen.

Nachstehend soll beschrieben werden, wie der Abstand zwischen Röntgenquelle 10 und dem Sensor 7 mittels eines Normals bestimmt wird, das im Ausführungsbeispiel aus einem Vierkugelnormal besteht, das vier auf den Ecken eines Quadrates angeordnete Kugeln umfasst.

- Die Abstände der Kugeln sind bekannt (kalibriert)
- Das Vierkugelnormal wird auf der Drehachse angeordnet
- Das Vierkugelnormal wird so eingedreht, dass die aufgespannte Ebene parallel zum Detektor steht
- Messung der vier Kugelpositionen im Bild an der Position Z1
- Berechnung der mittleren Vergrößerung M1 aus den vier gemessenen Kugeldistanzen
- den nominalen Kugeldistanzen und der nominalen Pixelgröße des Detektors
- Verfahren der Drehachse in Richtung der Quelle (oder Quelle und Detektor senkrecht zur Drehachse)
- Messung der vier Kugelpositionen im Bild an der Position Z2
- Berechnung der mittleren Vergrößerung M2 aus den vier gemessenen Kugeldistanzen
- den nominalen Kugeldistanzen und der nominalen Pixelgröße des Detektors
- Berechnung des Abstands Quelle Detektor nach folgender Gleichung:

$$AQD = dZ * M1 * M2 / ( M2 - M2 )$$

darin ist:

AQD: Abstand Quelle Detektor
M1: Vergrößerung an der Position Z1
M2: Vergrößerung an der Position Z2
dZ: Distanz zwischen den Position Z1 und Z2

- Berechnung der Distanz Quelle zu Z1 aus folgender Gleichung:

$$D1 = dZ * M2 / ( M1 - M2 )$$

- Berechnung der Distanz Quelle zu Z2 aus folgender Gleichung:

$$D2 = D1 + dZ = dZ * M1 / ( M1 + M2 )$$

- Berechnung der Position der Kegelachse auf dem Detektor nach folgender Gleichung

$$Pd = (Pkn1*D1 - Pkn2*D2) / dZ$$

darin ist:

Pd:     Abweichungsvektor der Kegelachs-Position von der Detektormitte
Pkn1:    Positionsvektor der Kugel n auf dem Detektor an der Position Z1
Pkn2:    Positionsvektor der Kugel n auf dem Detektor an der Position Z2

- Berechnung des mittleren Abweichungsvektors aus den vier Abweichungsvektoren für jede Kugelposition

Ein Verfahren zur Bestimmung der Y-Position des Drehachsenmittelpunktes unter Zugrundelegung ebenfalls eines Vierkugel-Normals mit an den Ecken eines Quadrates angeordneten Kugeln erfolgt wie nachstehend:

- Die Abstände der Kugeln sind bekannt (kalibriert)

- Das Vierkugelnormal wird auf der Drehachse angeordnet

- Das Vierkugelnormal wird so eingedreht, dass die aufgespannte Ebene parallel zum Detektor steht

- Messung der vier Kugelpositionen im Bild

- Berechnung der mittleren Vergrößerung M1 aus den vier gemessenen Kugeldistanzen,

- den nominalen Kugeldistanzen und der nominalen Pixelgröße des Detektors

- Drehung der Drehachse um 180°

- Messung der vier Kugelpositionen im Bild

- Berechnung der mittleren Vergrößerung M2 aus den vier gemessenen Kugeldistanzen,

- den nominalen Kugeldistanzen und der nominalen Pixelgröße des Detektors,

- Berechnung der Y Position des Drehmittelpunktes anhand der vier Kugelpositionen vor und nach der Drehung nach folgender Gleichung:

$$Pdyn = (Pkyn1 * M2 + Pkyn2 * M1) / (M1*M2)$$

darin ist:

Pdyn:    Y Position der Drehachse auf dem Detektor für Kugel n
Pkyn1:   Y Position der Kugel n bei Drehwinkel 0°
Pkyn2:   Y Position der Kugel n bei Drehwinkel 180'
M1:     mittlere Vergrößerung bei Drehwinkel 0°
M2:     mittlere Vergrößerung bei Drehwinkel 180°.

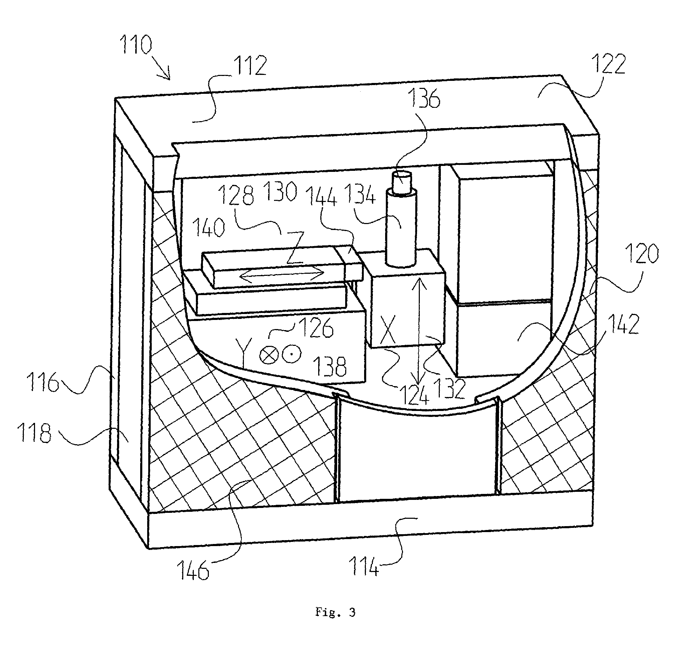

Den Fig. 3 bis 5 sind weitere eigenerfinderische Merkmale der erfindungsgemäßen Lehre zu entnehmen. Dabei ist in Fig. 3 ebenfalls rein prinzipiell ein Koordinatenmessgerät 110 mit einem Gehäuse 112 dargestellt, das eine Grundplatte 114, Rückwandung 116, Seitenwandungen 118, 120 sowie eine Kopfwandung 122 umfasst, die auch als Deckplatte zu bezeichnen ist.

Die x-Achse, y-Achse und z-Achse des Koordinatenmessgerätes sind in der Zeichnung mit dem Bezugszeichen 124, 126 und 128 gekennzeichnet. An der Innenseite 130 der Rückwandung 116 des Gehäuses 112 verläuft in x-Richtung einer Führung, entlang der, also in x-Richtung 124, eine Halterung 132 für einen Drehtisch 134 verstellbar ist, auf dem ein zu messendes Objekt 136 angeordnet ist. Mit anderen Worten ist auf der x-Achse 124 der Drehtisch 134 angeordnet. Ebenso kann die x-Achse mit dem Messobjekt auf dem Drehtisch angeordnet sein.

Entlang der y-Achse 126 verlaufen beispielhaft Führungen, entlang der eine Aufnahme 138 verschiebbar ist. Von der Aufnahme 138 geht entlang der z-Achse 128 verschiebbar eine Halterung 140 aus.

Von der Grundplatte 114 geht des Weiteren eine Röntgenquelle 142 aus, deren Röntgenstrahlung das auf dem Drehtisch 134 angeordnete Objekt 136 durchsetzen. Die Röntgenstrahlung wird ihrerseits von entsprechend geeigneten Sensoren wie CCD-Sensoren erfasst, die für Röntgenstrahlung empfindlich sind.

Des Weiteren können von der z-Achse 128, d. h. im Ausführungsbeispiel von der Halterung 140 Sensoren 144 ausgehen. Hierbei kann es sich um Sensoren handeln, die für Koordinatenmessgeräte üblich sind, also z. B. taktile oder optische Sensoren. Somit kann sowohl tomographiert werden als auch taktil oder optisch wie mit Bildverarbeitungssensoren, Laserabstandssensoren etc. gemessen werden.

Durch den Einsatz von Röntgenstrahlen ist es erforderlich, dass das Koordinatenmessgerät 110 im hinreichenden Umfang nach außen hin abgeschirmt ist. Hierzu ist erfindungsgemäß vorgesehen, dass zumindest einige der tragenden Bauelemente Abschirmfunktion ausüben. So kann z. B. die Grundplatte 114 und/oder die Rückwand 116 derart dimensioniert bzw. ausgebildet sein, dass die erforderliche Abschirmfunktion sichergestellt ist.

Die entsprechenden Wandungen 114, 116 üben dabei gleichzeitig die Funktion aus, die für den messtechnischen Aufbau erforderlich ist, nämlich im Ausführungsbeispiel Führung für die x- und y-Achse.

Zusätzlich besteht die Möglichkeit, Wandungen, die nicht die hinreichende Abschirmwirkung aufweisen, mit strahlungsdämmenden Schichten 146 innen- und/oder außenflächenseitig zu versehen. Hierbei handelt es sich insbesondere um Bleiblech.

Bezüglich der tragenden Wandungen, insbesondere derjenigen, die Abschirmfunktion ausüben, sollten bevorzugterweise Hartgestein wie Granit oder entsprechende Materialien zum Einsatz gelangen. Auch ist ein künstliches Hartgestein wie Polymerbeton denkbar, das im erforderlichen Umfang mit Röntgenstrahlen absorbierendem Material wie Magneteisenstein oder ähnliches versetzt sein kann.

Erfindungsgemäß übt das Gehäuse 112 des Koordinatenmessgerätes 110 bzw. Teile dieses eine Doppelfunktion aus, nämlich die der erforderlichen Abschirmung sowie die von Funktionsbauteilen des messtechnischen Aufbaus. Somit ergibt sich eine kompakte Konstruktion.

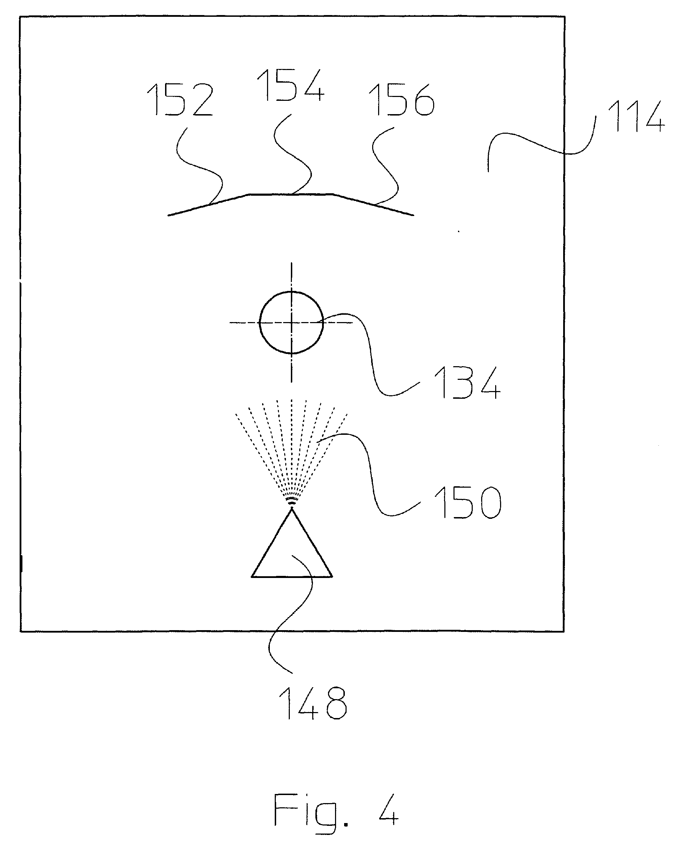

Um eine hohe Messdichte zu erreichen bzw. in jeweiliger Messposition nur geringe Bestrahlungszeiten in Kauf nehmen zu müssen, ohne dass Einbußen hinsichtlich der Messgenauigkeit erfolgen, ist entsprechend der Darstellung der Fig. 4 vorgesehen, dass gleichzeitig - also in jeder Messposition des Objekts 136 - mehrere Tomogramme unter unterschiedlichen Durchstrahlungswinkeln aufgenommen wird. So geht in Fig. 4 entsprechend den Ausführungen gemäß Fig. 3 von der Grundplatte 114 der Drehtisch 134 aus, auf dem ein nicht dargestelltes zu messendes Objekt angeordnet ist, das von von einem Röntgenstrahler 148 stammender Röntgenstrahlung 150 durchstrahlt wird. Die Strahlung wird im Ausführungsbeispiel von insgesamt drei Röntgensensoren 152, 154, 156 erfasst, so dass sich drei Tomogramme für unterschiedliche Durchstrahlungsrichtungen in einer Messposition des Objektes ergeben. In jeder Messposition, also jeder Winkelstellung des Drehtisches 34 werden die Sensoren 152, 154, 156 ausgelesen und Projektionsbilder für das Tomogramm gewonnen. Dabei ist die Winkelstellung der Sensoren 152, 154, 156 derart ausgelegt, dass sich die Winkel zwischen den Sensoren 152, 154, 156 jeweils um ganzzahlige Vielfache des beim Betreiben des Computer-Tomographen verwendeten Winkelschritts des Drehtisches 136 unterscheiden, wobei der zweite und der dritte Sensor 154, 156 zum vorausgehenden ersten Sensor 152 bzw. zweiten Sensor 154 um ein Drittel des Winkelschritts verdreht angeordnet sind.

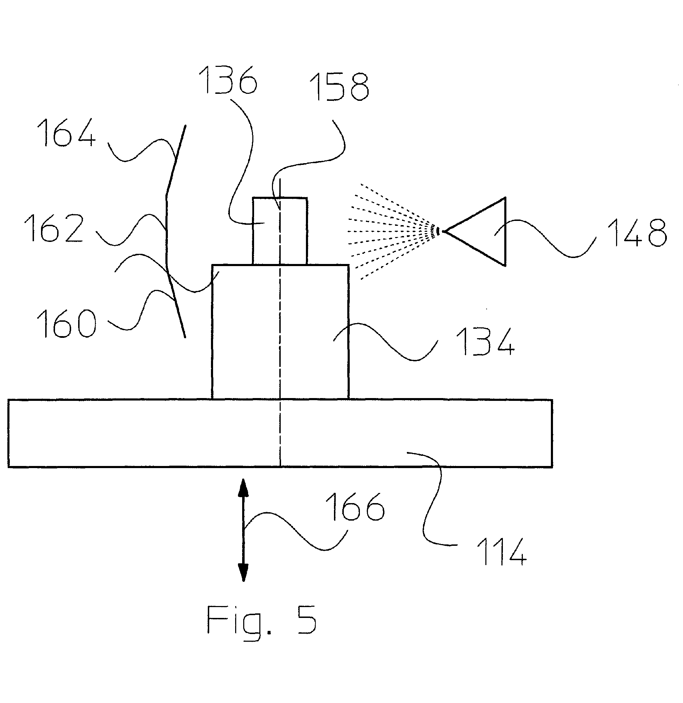

Um mehrere Tomogramme des zu messenden Objekts 136 aufzunehmen, wobei der Winkel zwischen Drehachse 158 des Drehtisches 154 und Röntgenstrahlung 150 scheinbar verändert wird, sind im Ausführungsbeispiel der Fig. 5 beispielhaft drei Sensoren 160, 162, 164 in unterschiedlichen Winkeln zu der Hauptstrahlungsrichtung der Röntgenstrahlungsquelle 148 angeordnet, wodurch das scheinbare Verschwenken der Röntgenstrahlungsquelle zur Drehachse 158 simuliert wird.

Der in Fig. 5 eingezeichnete Doppelpfeil 166 soll symbolisieren, dass der Drehtisch 134 entlang der Drehachse 158 parallel zur X-Achse verstellbar ist.

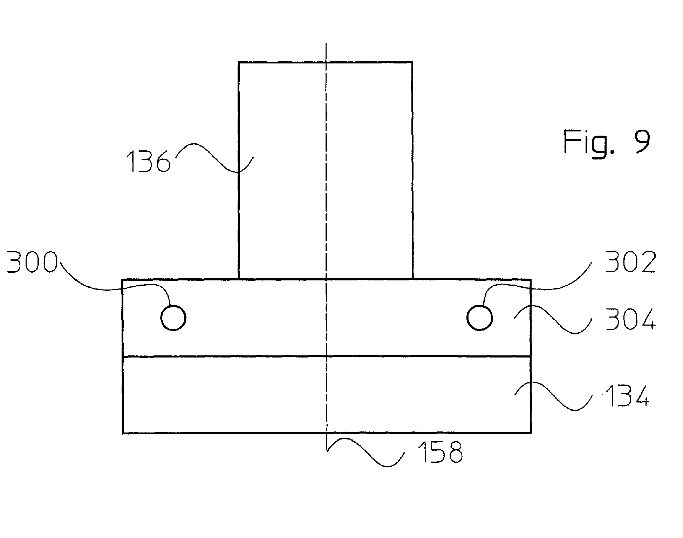

Wie anhand der Fig. 9 prinzipiell verdeutlicht wird, kann beim Tomographieren grundsätzlich ein Kalibrierkörper vorzugsweise in Form von Kugeln 300, 302 mit tomographiert werden, wodurch sich die Relativlage der Drehachse 158 des Drehtisches 134 ergibt, auf dem das zu messende Objekt 136 angeordnet ist. Die Kugeln 300, 302 können in einer Aufnahme 304 angeordnet sein, die gegenüber Röntgenstrahlen eine geringe Absorption zeigt, wohingegen die Kugeln 300, 302 stark absorbierend sind und z. B. aus Stahl bestehen. Hierdurch kann problemlos während des Tomographierens die Position der Drehachse 158 zum Koordinatenmessgerät bzw. zur Röntgenquelle 10 bzw. zum Sensor 7 bestimmt und anschließend mathematisch korrigiert werden.

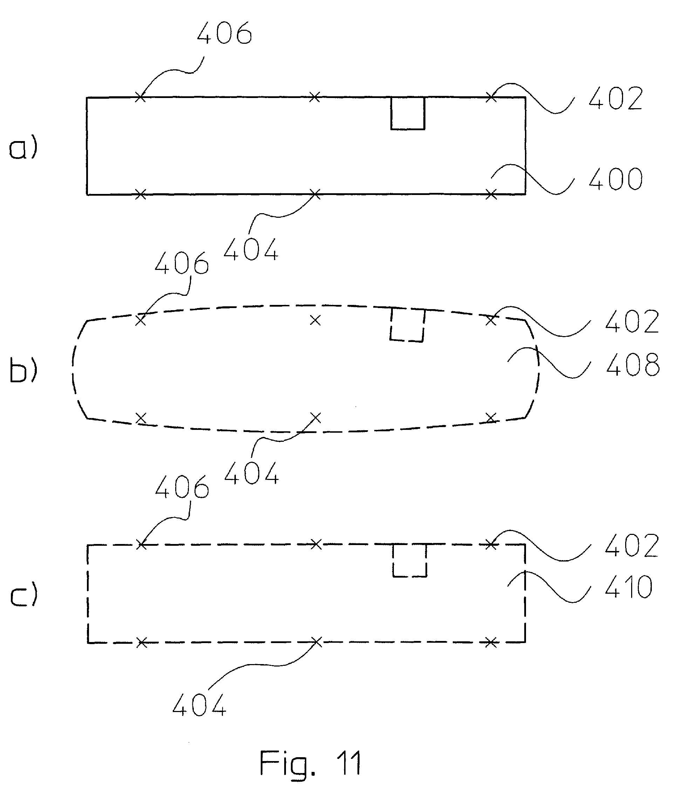

Erfindungsgemäß werden in dem Koordinatenmessgerät mittels taktiler und/oder optischer Sensorik Messpunkte am Messobjekt aufgenommen und zur Korrektur der mit der Röntgensensorik ermittelten Messpunkte herangezogen. Dies soll auch anhand der Fig. 11 verdeutlicht werden. Dieser ist das Prinzip eines entsprechenden Korrekturverfahrens zu entnehmen. So ist in Fig. 11a ein Messobjekt 400 dargestellt, das an ausgewählten Punkten taktil und/oder optisch gemessen wird. Entsprechende Messpunkte sind beispielhaft mit den Bezugzahlen 402, 404, 406 gekennzeichnet. Beim Tomographieren, das anschließend im selben Koordinatenmessgerät stattfindet, erhält man die durch typische Fehler der Tomographie veränderte Gestalt in der tomographierten Punktwolke 408. Dies können z. B. tomographietypische Artefakte sein. Die tomographierten Messpunkte werden auf der Basis der zur Verfügung stehenden mit optischer und/oder taktiler Sensorik genauer gemessenen Messpunkte, die in Fig. 11b noch einmal eingezeichnet sind, in ihrer Lage korrigiert. Dabei kann zwischen den taktilen und optisch gemessenen Messpunkten eine Interpolation erfolgt sein. Als Ergebnis erhält man sodann eine geometrisch korrigierte tomographisch gemessene Punktwolke 410, die besser der Gestalt des Messobjekts 400 entspricht als die Originaldaten des Tomogramms. Dies zeigt ein Vergleich der Fig. 11b und 11c.

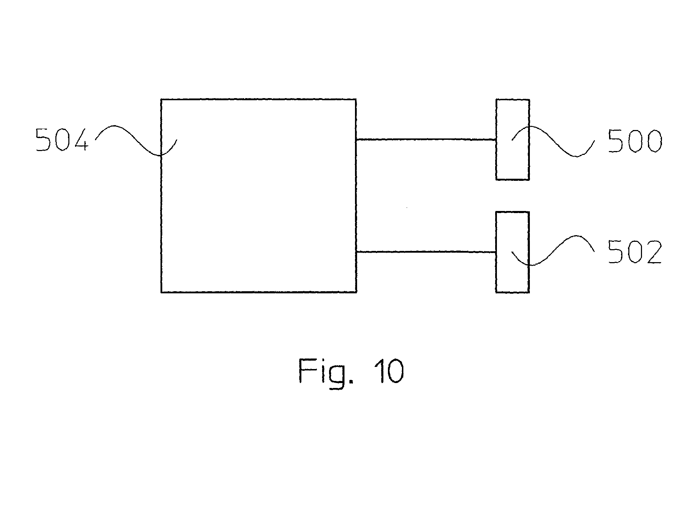

Bei der Durchführung der Messungen und der Auswertung der Messergebnisse kann die Bildverarbeitungssensorik für das Messen mit sichtbarem Licht im Durchlichtverfahren - gegebenenfalls auch im Auflichtverfahren - mit der gleichen Bildverarbeitungsauswerteeinheit bzw. dem gleichen Bildverarbeitungsboard wie die Röntgensensorik gekoppelt werden. Softwaregesteuert kann sodann zwischen den beiden Sensoren umgeschaltet und in der gleichen Hardware digitalisiert und gerechnet werden. Dies wird prinzipiell durch die Fig. 10 verdeutlicht, in der eine Bildverarbeitungssensorik 500 und eine Röntgensensorik 502 mit dem gleichen Bildverarbeitungsboard 504 verbunden sind, um in zuvor beschriebener Weise arbeiten zu können.

**Patentansprüche**

1. Verfahren zum Messen eines Objektes mit einem Koordinatenmessgerät mit einer Röntgensensorik umfassend eine Röntgenstrahlquelle und zumindest einen die Röntgenstrahlen erfassenden Röntgenstrahlensensor,
   **dadurch gekennzeichnet,**
   **dass** mehrere Ausschnitte des Messobjektes nacheinander durch die bekannten Verfahren der Tomographie erfasst werden und für die gesamte Menge der zusammengesetzten Durchstrahlungsbilder die 3D-Rekonstruktion erfolgt, wobei vorzugsweise größere Messobjekte als durch das Sehfeld des Tomographen bedingt gemessen werden.

2. Verfahren nach Anspruch 1,
   **dadurch gekennzeichnet,**
   **dass** die Röntgensensorik in x-, y- und/oder z-Richtung des Koordinatenmessgerätes relativ zu dem Objekt positioniert wird und zur Aufnahme eines Tomogramms benötigte Drehachse zum Drehen des Objekts auf einer Verfahrachse zur Vergrößerung des Messbereichs in Richtung der Drehachse angeordnet wird.

3. Verfahren nach Anspruch 1 oder 2,
   **dadurch gekennzeichnet,**
   **dass** das Koordinatenmessgerät mindestens mit einer zur Arbeitsrichtung (x-und/oder y-Richtung) der Röntgensensorik senkrechten Achse (Z-Achse) ausgestattet wird, wobei die Drehachse vorzugsweise um eine vertikal verlaufende Achse gedreht wird.

4. Verfahren nach einem der Ansprüche 1 bis 3,
   **dadurch gekennzeichnet,**
   **dass** Wirkungsachse bzw. mechanische Achse für die Röntgensensorik bzw. deren Sensor horizontal und/oder senkrecht zur Rotationsachse ausgerichtet wird.

5. Verfahren nach zumindest einem der Ansprüche 1 bis 4,
   **dadurch gekennzeichnet,**
   **dass** Strahlungsquelle für die Röntgensensorik synchron mit dem zugeordneten Sensor bewegt werden oder dass die Strahlungsquelle für die Röntgensensorik im Koordinatensystem des Koordinatenmessgerätes fest angeordnet wird.

6. Verfahren nach zumindest einem der Ansprüche 1 bis 5,
   **dadurch gekennzeichnet,**
   **dass** der Röntgensensor bzw. die Röntgensensorik in mindestens einer Achse zum Objekt verstellbar angeordnet wird.

7. Verfahren nach zumindest einem der Ansprüche 1 bis 6,
   **dadurch gekennzeichnet,**
   **dass** eine Messbereichsanpassung durch Verstellen des Abstandes zwischen Röntgensensor und Strahlungsquelle und/oder durch relatives Verstellen von Objekt und Röntgensensorik zueinander erfolgt.

8. Verfahren nach vorzugsweise einem der Ansprüche 1 bis 7,
   **dadurch gekennzeichnet,**
   **dass** mittels translatorischer Relativbewegung zwischen Objekt und Röntgenstrahlungsquelle bzw. Röntgensensor ein Bereich aufgenommen wird, der größer als Sensorfläche ist.

**9.** Koordinatenmessgerät (110) zum Messen eines Objektes (3, 136) mit einer Röntgensensorik umfassend eine Röntgenstrahlenquelle (10) und zumindest einen die Röntgenstrahlen erfassenden Röntgenstrahlensensor (7),
**dadurch gekennzeichnet,**
**dass** die Röntgensensorik (7, 10) in dem Koordinatenmessgerät (10) in x-, y-und/oder z-Richtung zu dem Objekt positionierbar ist und dass Drehachse (18) des Objekts (3) auf einer Verfahrachse (1) zur Erweiterung des Messbereichs entlang der Drehachse angeordnet ist.

**10.** Koordinatenmessgerät nach Anspruch 9,
**dadurch gekennzeichnet,**
**dass** das Koordinatenmessgerät (110) mindestens mit einer zur Arbeitsrichtung (y- und/oder z-Achsenrichtung) der Röntgensensorik (7, 10) senkrechten Drehachse (18; x-Achsenrichtung) ausgestattet ist, wobei die Drehachse (18, 158) vorzugsweise um eine vertikal verlaufende Achse (x-Achse) rotiert.

**11.** Koordinatenmessgerät nach Anspruch 9 oder 10,
**dadurch gekennzeichnet,**
**dass** die Strahlenachse für den Röntgensensor (8) horizontal und/oder senkrecht zur Drehachse (18, 138; x-Achse) ausgerichtet ist.

**12.** Koordinatenmessgerät nach zumindest einem der Ansprüche 9 bis 11,
**dadurch gekennzeichnet,**
**dass** Strahlungsquelle (10, 11) für die Röntgensensorik synchron mit dem zugeordneten Sensor (7, 8) bewegbar ist oder dass die Strahlungsquelle (10) für die Röntgensensorik im Koordinatensystem des Koordinatenmessgerätes (110) fest angeordnet ist.

**13.** Koordinatenmessgerät nach zumindest einem der Ansprüche 9 bis 12,
**dadurch gekennzeichnet,**
**dass** die Röntgensensorik (7, 10) in mindestens einer Achse zum Objekt (3, 136) verstellbar angeordnet ist.

**14.** Koordinatenmessgerät nach zumindest einem der Ansprüche 9 bis 13,
**dadurch gekennzeichnet,**
**dass** eine Messbereichsanpassung durch Verstellen des Abstandes zwischen dem Röntgensensor (7) und der Strahlungsquelle (10) und/oder durch relatives Verstellen der Röntgensensorik (7, 10) zu dem Objekt (3) vorzugsweise automatisch erfolgt.

**15.** Koordinatenmessgerät nach zumindest einem der Ansprüche 9 bis 14,
**dadurch gekennzeichnet,**
**dass** der Röntgensensor (7) auf separater Verfahrachse bzw. mechanischer Achse angeordnet ist.

Fig. 1

Fig. 2

Fig. 3

Fig. 4

Fig. 5

Fig. 6

Fig. 7

Fig. 8

136

Fig. 9

300

302

304

134

158

Fig. 10

a)

406

402

400

b)

406 404

402

408

c)

406 404

402

410

404

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10331419 A
- DE 10044169 A
- DE 3806686 C
- EP 1389263 A
- US 20030043964 A
- DE 10001239 A
- DE 4445331 A
- EP 0504609 A
- US 5038378 A

**In der Beschreibung aufgeführte Nicht-Patentliteratur**

- *Die Bibliothek der Technik,* vol. 248